# EUROPÄISCHE PATENTSCHRIFT

(11) **EP 3 693 152 B1**
(45) Veröffentlichungstag und Bekanntmachung des Hinweises auf die Patenterteilung: **08.04.2026**
(21) Anmeldenummer: 20154991.2
(22) Anmeldetag: 31.01.2020
(51) Int. Cl.: B29C 64/194, B33Y 10/00, B33Y 80/00, B25J 13/08, B25J 19/02, B25J 19/00, B25J 15/08, B25J 15/00, A61F 2/58

(54) **VERFAHREN ZUR HERSTELLUNG EINES GREIFBACKENS MITTELS 3D-DRUCK**
METHOD FOR PRODUCING A GRIPPER BY MEANS OF 3D PRINTING
PROCÉDÉ DE FABRICATION D'UN PRÉHENSEUR PAR IMPRESSION 3D

(30) Priorität: 06.02.2019 DE 102019102913
(43) Veröffentlichungstag der Anmeldung: 12.08.2020
(73) Patentinhaber: Hochschule Offenburg, 77652 Offenburg (DE)
(72) Erfinder: Hangst, Nikolai, 78655 Dunningen/Seedorf (DE); Junk, Stefan, 66123 Saarbrücken (DE); Wendt, Thomas, 79263 Simonswald (DE)
(74) Vertreter: Eder Schieschke & Partner mbB

(56) Entgegenhaltungen:
- EP-A1- 3 297 580
- EP-B1- 3 297 580
- WO-A1-2020/041221
- US-A1- 2013 331 949
- LEE JU-KYOUNG ET AL: "Development of Direct-printed Tactile Sensors for Gripper Control through Contact and Slip Detection", INTERNATIONAL JOURNAL OF CONTROL, AUTOMATION AND SYSTEMS, KOREAN INSTITUTE OF ELECTRICAL ENGINEERS, SEOUL, KR, vol. 16, no. 2, 1 March 2018 (2018-03-01), pages 929 - 936, XP036476307, ISSN: 1598-6446, [retrieved on 20180301], DOI: 10.1007/S12555-017-0151-X

## Beschreibung

Die Erfindung betrifft ein Verfahren zur Herstellung eines Roboterelements, nämlich eines Greifbackens,

An Greifsysteme in der Robotik werden immer höhere Anforderungen mit sich erweiternden Anwendungsprofilen gestellt. Konventionelle Herstellungsarten, wie beispielsweise Gießen mittels entsprechender Formen, spanende Verfahren, etc., decken jedoch die Anwendungsgebiete nur teilweise ab, so dass nicht nur Greifsysteme an einem Roboter gewechselt werden müssen und die Rüstzeit des Roboters erhöhen, sondern auch entsprechend mehrere unterschiedliche Greifsysteme hergestellt werden müssen.

Der vorliegenden Erfindung liegt daher die Aufgabe zugrunde, ein Verfahren zur Herstellung eines Roboterelements, nämlich eines Greifbackens, zu schaffen, der multifunktional anwendbar ist und die Rüstzeit eines Roboters reduziert.

Diese Aufgabe wird erfindungsgemäß durch ein Verfahren mit den Merkmalen des Anspruchs 1 gelöst.

Dadurch, dass ein ganzer Greifbacken mittels 3D-Druck gefertigt wird, entfallen sonst erforderliche weitere Herstellungsprozesse, wie beispielsweise Anbringen von separaten Sensoren und Beschichtung mit (meist flexiblen) Materialen.

Erfindungsgemäß erfolgt die Herstellung eines derartigen Greifbackens mittels Multimaterialdruck, so dass nicht nur besondere, meist flexible Außenflächen bzw. -schichten eines Greifelements flexibel gestaltet werden können, sondern auch wenigstens ein Sensor (kapazitiv, induktiv, resistiv, Hall-Effekt) während der Herstellung des Greifbackens mit integriert und während des Drucks des Greifbackens mitgedruckt, also selbst mittels Druck hergestellt wird.

Zudem bietet das erfindungsgemäße Herstellungsverfahren die Möglichkeit, eine erforderliche Belastbarkeit, Reißfestigkeit, Biegefestigkeit, Steifigkeit, Härte, etc. durch entsprechende Materialwahl, insbesondere der unteren bzw. inneren Schichten des Greifelements zu gewährleisten, wobei zur Erzeugung unterschiedlicher Schichten unterschiedliche Materialen verwendet werden können. Auf diese Weise können gewünschte unterschiedliche Materialeigenschaften in ein und demselben Element bzw. Bauteil in einem einzigen Herstellungsverfahren integriert werden, ohne dass weitere Bearbeitungsschritte erforderlich sind.

In bevorzugter Ausgestaltung der Erfindung wird eine Kombination von mehreren (gleichartigen oder unterschiedlichen) Sensoren während des Drucks des Greifbackens mitgedruckt. Auf diese Weise werden redundante und/oder multifunktionale Sensoriken ermöglicht, ohne dass Nachbearbeitungen eines erfindungsgemäß hergestellten Bauteils erforderlich sind.

In weiterer Ausgestaltung der Erfindung werden Anschlüsse, Leitungen, Stecker und/oder Verbindungselemente mitgedruckt, ohne dass Nachbearbeitungen eines erfindungsgemäß hergestellten Bauteils erforderlich sind.

In besonders bevorzugter Ausgestaltung der Erfindung wird Verbundmaterial, insbesondere Faserverstärkung, mitgedruckt (Mikrofaser) oder ebenfalls während des Drucks eingebettet (Endlosfaser). Beispielsweise können sich sehr kurze Faseranteile, sogenannte Mikrofasern (insbesondere Kohlenstofffaser, Polyamid, etc.), bereits im Druckmaterial befinden und auf diese Weise mittels einer Düse auf das Druckbett bzw. auf die bereits vorhandenen Schichten aufgebracht (mitgedruckt) werden. Andererseits kann eine Faser auch als Endlosfaser (insbesondere Kohlenstofffaser) mithilfe einer zweiten Düse in jede beliebige Schicht während des Drucks oder während einer Druckpause eingelegt werden.

In weiterer Ausgestaltung der Erfindung werden Sensoren und Faserverstärkung während des Drucks auf unterschiedliche Ebenen verteilt. Hierdurch kann verhindert werden, dass leitfähige Faserverstärkungen Sensoren beeinträchtigen oder gar Kurzschlüsse erzeugen. Hierbei kann an einem Übergang zwischen einer Schicht bzw. Schichten, welche Sensoren enthalten und Schichten mit elektrisch leitfähigen Fasern zusätzlich eine Isolierschicht vorgesehen sein, so dass eine (elektrisch) funktionale Beeinträchtigung eines Sensors ausgeschlossen werden kann.

In weiterer Ausgestaltung der Erfindung wird wenigstens eine Kamera durch Einlegen/Einbetten während des Drucks (eventuell auch während einer Pause vor Drucken der nächsten Schicht) integriert.

In besonders vorteilhafter Ausgestaltung der Erfindung wird eine gesamte Greifbacke eines Roboters in einem einzigen (Druck-) Stück, eventuell inklusive Gelenkelemente, mittels 3D-Multimaterialdrucks gedruckt.

Durch die vorgenannten Ausgestaltungen kann vorteilhafterweise auch das Gewicht im Vergleich zu herkömmlichen Greifsystemen oder Betätigungssystemen verringert werden, da Elemente wie Sensoren, Leitungen, Stecker, Verbindungselemente, etc. mitgedruckt und/oder eingebettet werden. Auch die erfindungsgemäße Faserverstärkung mittels 3D-Multimaterialdruck trägt zur Verringerung des Gewichts bei, indem eine höhere Belastbarkeit, Reißfestigkeit, Biegefestigkeit, Steifigkeit, Härte, etc. des Materials durch vorgenannte Faserverstärkung statt durch Erhöhung der Materialmenge erreicht wird. Zudem können durch das erfindungsgemäße Verfahren auf einfache Weise bionische Formen realisiert werden, d.h. Material wird nur dort verwendet, wo es, beispielsweise aus Festigkeitsgründen, auch notwendig ist.

Weitere vorteilhafte Ausgestaltungen der Erfindungen sind in den abhängigen Ansprüchen angegeben.

Die Erfindung wird nachfolgend anhand in der Zeichnung angegebener Ausführungsformen näher erläutert.

In der Zeichnung zeigen:
- Fig. 1a: eine Vorderansicht einer schematischen Darstellung eines nach der Erfindung hergestellten Greifsystems eines Roboters;
- Fig. 1b: eine Seitenansicht auf ein Greifsystem nach Fig. 1a;
- Fig. 1c: eine Schnittansicht entlang der Schnittlinie A-A in Fig. 1b;
- Fig. 1d: eine vergrößerte Ansicht des Details Z in Fig. 1a;
- Fig. 2a: eine Seitenansicht der Greiffinger aus Fig. 1a bis Fig. 1d mit kapazitiven Sensoren;
- Fig. 2b: eine Schnittansicht des Greiffingers entlang der Linie A-A in Fig. 2a;
- Fig. 3a: eine Seitenansicht der Greiffinger aus Fig. 1a bis Fig. 1d mit induktiven Sensoren;
- Fig. 3b: eine Schnittansicht des Greiffingers entlang der Linie A-A in Fig. 3a;
- Fig. 4a: eine Seitenansicht der Greiffinger aus Fig. 1a bis Fig. 1d mit resistiven Sensoren;
- Fig. 4b: eine Schnittansicht des Greiffingers entlang der Linie A-A in Fig. 4a;
- Fig. 5a: eine Seitenansicht der Greiffinger aus Fig. 1a bis Fig. 1d mit Hall-Sensoren;
- Fig. 5b: eine Schnittansicht des Greiffingers entlang der Linie A-A in Fig. 5a;
- Fig. 6a: eine Seitenansicht der Greiffinger aus Fig. 1a bis Fig. 1d mit einer Kombination aus kapazitiven, induktiven und resistiven Sensoren;
- Fig. 6b: eine Schnittansicht des Greiffingers entlang der Linie A-A in Fig. 6a;
- Fig. 7a: eine Seitenansicht der Greiffinger aus Fig. 1a bis Fig. 1d mit kapazitiven, induktiven, resistiven Sensoren sowie Hall-Sensoren jeweils in unterschiedlichen Schichten;
- Fig. 7b: Schnittansichten des Greiffingers entlang der Linie A-A, B-B, C-C und D-D in Fig. 7a;
- Fig. 8a: eine Seitenansicht der Greiffinger aus Fig. 1a bis Fig. 1d mit Multisensoren;
- Fig. 8b: eine Schnittansicht des Greiffingers entlang der Linie A-A in Fig. 6a;

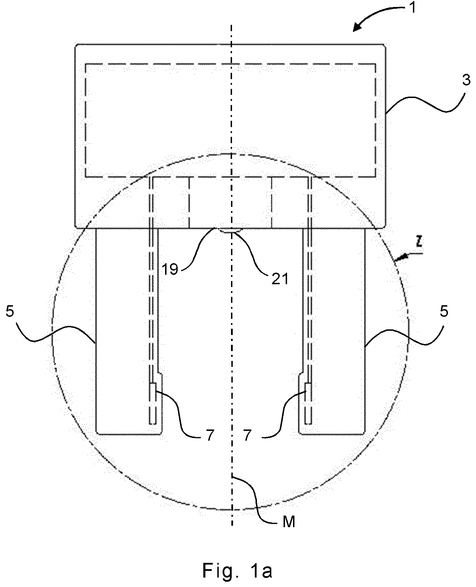

Das in Fig. 1a bis Fig. 1d dargestellte Greifsystem 1 eines Roboters besteht aus einer Steuerungseinheit 3 und mehreren, beispielsweise zwei, als Finger 5 ausgebildeten Greifelementen oder Betätigungselementen. Die Steuerungseinheit 3 bildet dabei das mechanische Bindeglied zwischen Fingern 5 und Roboter und stellt vorzugsweise zudem eine elektrische Verbindung zwischen beiden Komponenten her. Jeder Finger 5 ist vorzugsweise mit einem eigenen Aktor versehen, um ein beliebiges Verdrehen der Finger 5 sowie deren schnellen Aus- und Umbau zu gewährleisten. Zusätzlich können die Finger vorzugsweise - um beispielsweise 7° - zur Längsachse L in beide Richtungen geneigt werden, um eine Reduzierung des Greifweges bzw. der Greifzeit bei unterschiedlichen Gegenständen zu ermöglichen.

Zum Greifen können die vorzugsweise zur Mittelachse M des Greifsystems 1 rotationssymmetrisch ausgebildeten Finger 5 entlang einer Achse quer zur Mittelachse des Greifsystems 1 (horizontal in der Zeichenebene der Fig. 1a) zwischen einem maximalen Abstand (wie in der Zeichnung dargestellt) und einem minimalen Abstand (bis hin zur gegenseitigen Berührung der Finger) zueinander bewegt werden.

Selbstverständlich steht das dargestellte Greifsystem 1 mit zwei Fingern 5 nur exemplarisch für allgemeine Greifsysteme nach der Erfindung, wobei auch Greifsysteme mit mehreren Fingern, beispielsweise drei Fingern radial um die Mittelachse M gleich verteilt, denkbar sind.

Die Drehung, Neigung und Bewegung der Finger 5 kann hierbei durch eine entsprechende Vorrichtung (insbesondere elektrische Stellmotoren) im Bereich der Verbindung zwischen der Steuerungseinheit 3 und den Fingern 5 oder in den Fingern 5 selbst (beispielsweise in unterschiedlichen Fingerabschnitten oder zusätzlich vorhandenen Fingergelenken) realisiert sein.

Die zwei in Fig. 1a bis Fig. 1d dargestellten Finger 5 weisen an ihren Innenseiten Sensoren 7 auf, welche in einer der oberen Schichten 23 oder in der obersten Schicht in Richtung der Mittelachse M (mittels Multimaterialdruck) integriert sind und vorzugsweise unterhalb der als Außenhaut fungierenden äußersten Schicht liegen. Auf diese Weise sind die Sensoren 7 gegenüber einem äußeren mechanischen Kontakt geschützt. Als Arten von Sensoren kommen hierbei beispielsweise kapazitive, resistive, induktive Sensoren wie auch Hall-Sensoren in Frage.

Die Sensoren 7 weisen Zuleitungen 7a auf, die innerhalb der Finger 5 ebenso wie die Sensoren 7 mitgedruckt werden und zum Steuerungssystem bzw. der darin untergebrachten Elektronik führen. Die Sensoren 7 sind über deren Zuleitungen 7a in nicht näher dargestellter Weise gegebenenfalls über entsprechende Verbindungselemente (Stecker, Buchsen, etc.) von außen zugänglich und an entsprechende Anschlüsse eines Roboters anschließbar. Hierbei können die Verbindungselemente ebenfalls mitgedruckt oder eingebettet werden.

Der Sensor 7 liegt in seiner unterschiedlichen Ausbildung (Kondensator, Spule, Widerstand, Dehnungsmessstreifen, Hall-Sensor, etc.) vorzugsweise koplanar innerhalb einer (beispielsweise der oberen äußeren) Schicht, die selbst als elektrisch isolierende Schicht ausgebildet ist.

Unterhalb oder auch oberhalb dieser Schicht mit einem Sensor 7 können dagegen auch Faserverstärkungen (durch Mikrofaser im Druckmaterial oder Einbringen von Endlosfasern in das Druckbett) vorhanden sein, selbst wenn diese aus einem elektrisch leitfähigen Material (z.B. Kohlenstoff) bestehen.

Im mittleren Bereich zwischen den Fingern 5 liegend ist in der Steuerungseinheit 3 eine Kamera 19 eingebettet, deren Objektiv 21 in Richtung der Finger 5 bzw. in Richtung eines damit zu greifenden Objekts gerichtet ist und den Bereich zwischen den Fingern 5 abdeckt, um ein zu greifendes oder zu betätigendes Objekt bildlich zu erfassen.

Die Kamera 19 kann während einer Druckunterbrechung eingebettet werden, indem sie in eine gedruckte Ausnehmung eingelegt oder um eine positionierte Kamera herum gedruckt wird, so dass sich deren Linse oder Objektiv an einer gewünschten Stelle mit gewünschtem Fokus befindet.

Vorteilhafterweise werden hierbei die Verbindungsleitungen (Anschlüsse) und Verbindungselemente zum Anschließen der Kamera mitgedruckt oder ebenfalls eingebettet, so dass eine Kontaktierung der Kamera nicht von außen bzw. über Außenflächen erfolgen muss. Eine derartige integrierte Bauweise trägt dazu bei, die Bauhöhe zu verringern und unerwünschte äußerliche Kabelführungen zu vermeiden.

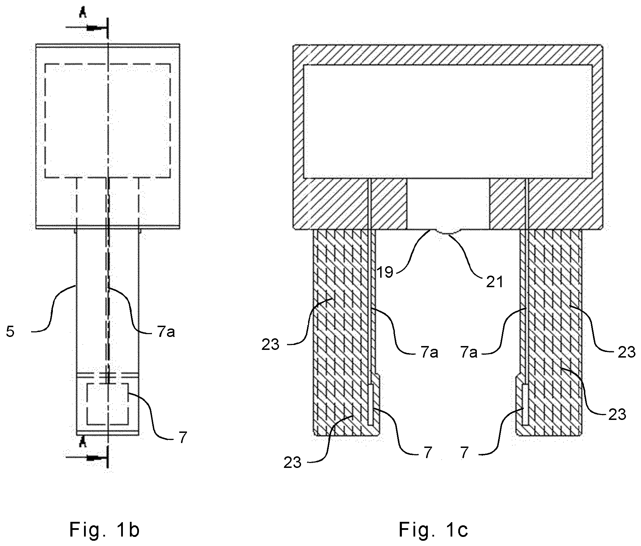

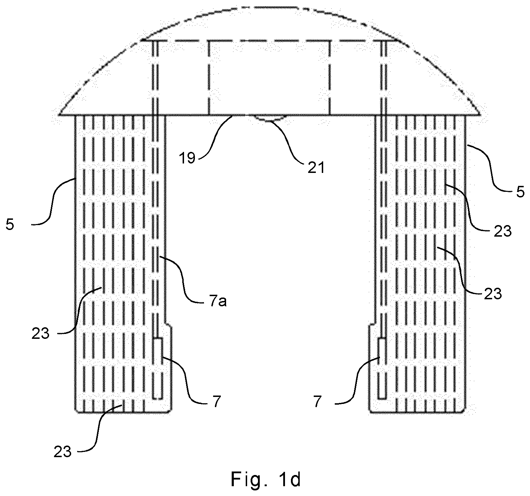

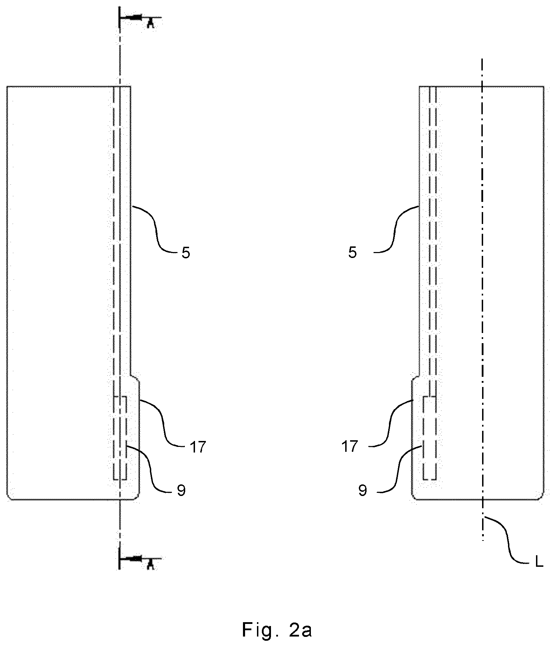

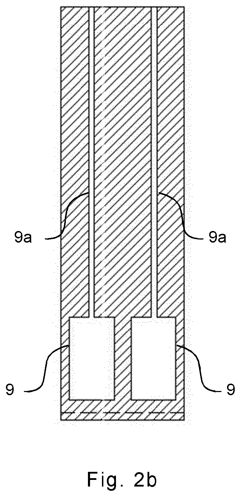

In Fig. 2 bis Fig. 8 werden unterschiedliche Ausgestaltungen des in Fig. 1 bzw. in den Figuren 1a bis 1d allgemein dargestellten Sensors 7 erläutert.

So weisen die in Fig. 2a und Fig. 2b als Einzelheit dargestellten Finger 5 in ihren Endbereichen bzw. Greifflächen der Fingerspitzen in einem ersten Ausführungsbeispiel kapazitive Sensoren 9 mit Zuleitungen 9a auf. Wie aus Fig. 2a ersichtlich, liegen die beiden Platten der Sensoren 9 in der Zeichenebene horizontal nebeneinander, wobei selbstverständlich auch eine Anordnung übereinander (vertikal in der Zeichenebene) denkbar ist. Die beiden Platten des hierdurch realisierten kapazitiven Sensors (Kondensators) liegen koplanar in derselben (äußeren) Schicht, die zudem als Isolierung (Dielektrikum) zwischen den Platten dient.

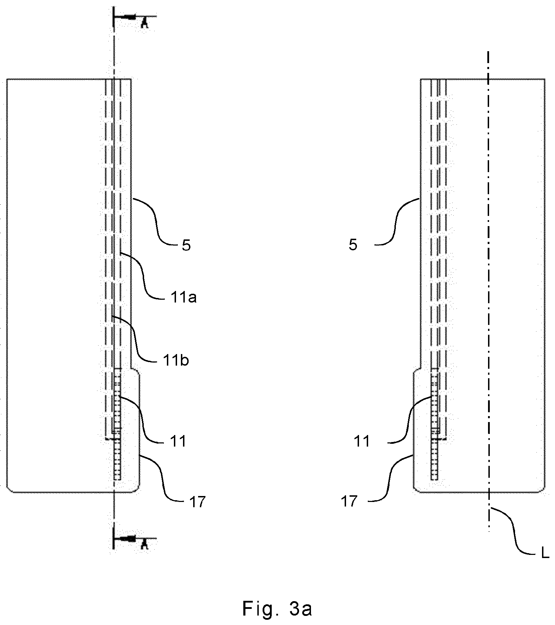

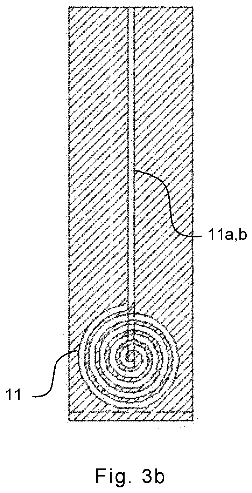

In dem in Fig. 3a und Fig. 3b dargestellten zweiten Ausführungsbeispiel weisen die Finger 5 innerhalb einer Schicht (koplanar zur Schichtebene) befindliche Spulen 11 als induktive Sensoren (statt kapazitiver Sensoren 9) mit Zuleitungen 11a (Endabgriff) und 11b (Mittelabgriff) auf. Bei der in Draufsicht dargestellten Schicht handelt es sich um eine beliebige Schicht, welche induktiv beeinflusst werden kann. Vorzugsweise handelt es sich um eine der oberen Schichten oder gar um die äußerste Schicht des Fingers, um die Empfindlichkeit des Sensors zu erhöhen.

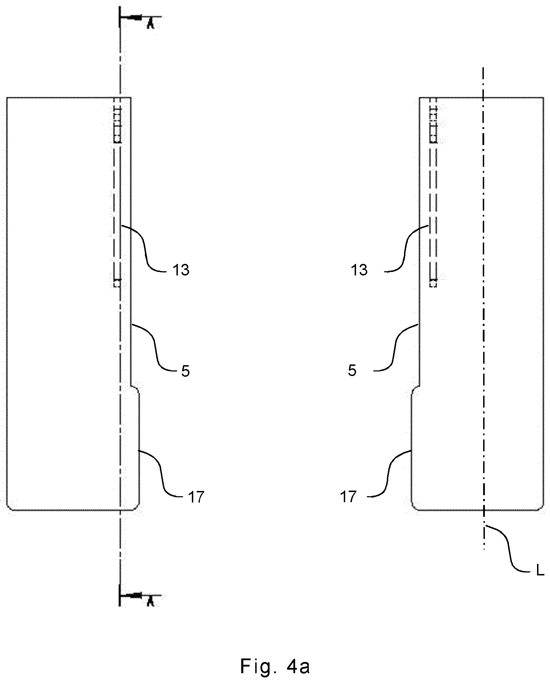

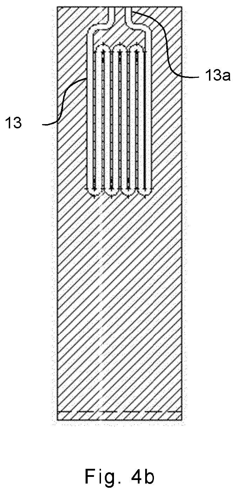

Das in Fig. 4a und Fig. 4b dargestellte dritte Ausführungsbeispiel zeigt, wie in analoger Weise zu Fig. 3a und Fig. 3b statt Spulen 11 Dehnungsmessstreifen 13 (resistiver Sensor) koplanar mit Zuleitungen 13a in einer Ebene gedruckt wurden, wobei es sich auch hier bei der dargestellten Schicht um eine beliebige Schicht handeln kann, welche in diesem Fall mechanisch beeinflusst wird, und nicht zwingend um die äußerste Schicht handeln muss. Der Dehnungsmessstreifen 13 weist in Richtung der Längsachse L des Fingers 5 vorzugsweise ein größere Ausdehnung (Länge) als Breite quer zur Längsachse L auf, so dass Längenänderungen infolge einer unter Last erfolgten Biegung des Fingers 5 leichter detektiert werden können.

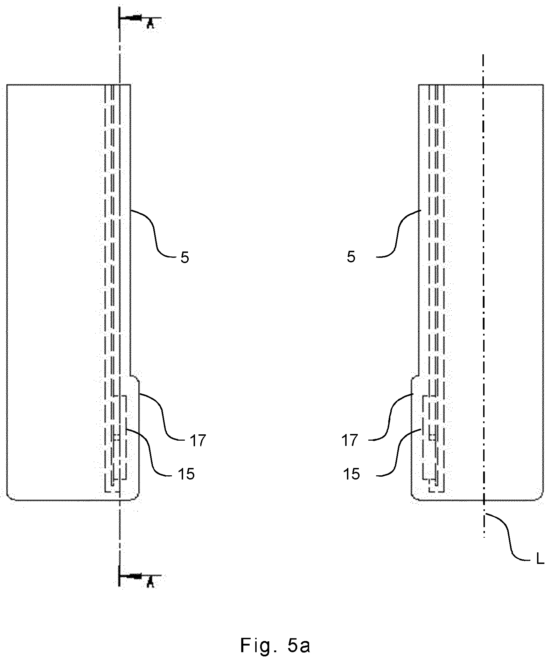

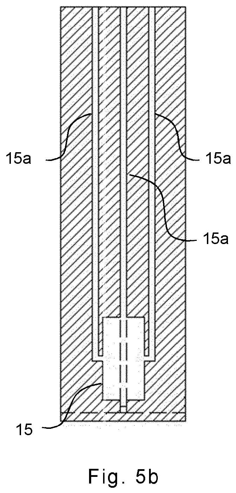

Das in Fig. 5a und Fig. 5b dargestellte vierte Ausführungsbeispiel zeigt, wie - in analoger Weise zu Fig. 4a und Fig. 4b statt Dehnungsmessstreifen 13 - Hall-Sensoren 15 koplanar mit Zuleitungen 15a, b, c in einer Ebene gedruckt wurden, wobei es sich auch hier bei der dargestellten Schicht um eine beliebige Schicht handeln kann, welche in diesem Fall magnetisch (induktiv) beeinflusst wird, und nicht zwingend um die äußerste Schicht handeln muss.

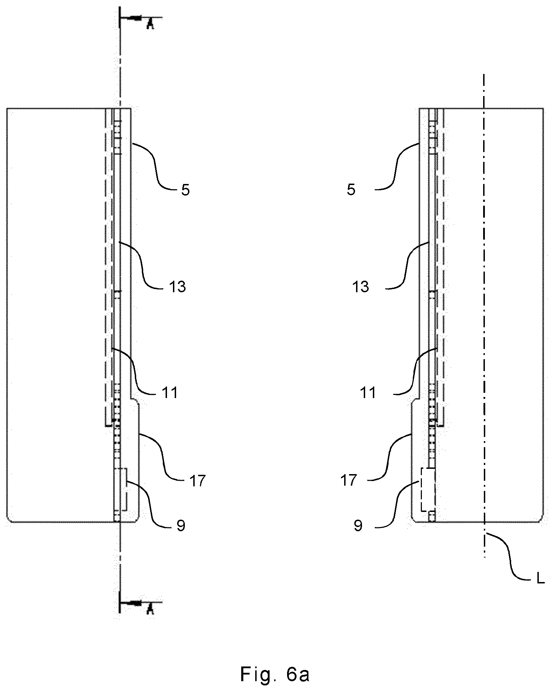

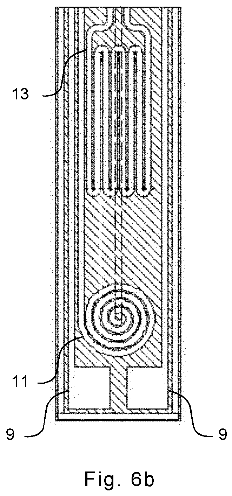

Aus dem in Fig. 6a und Fig. 6b dargestellten fünften Ausführungsbeispiel ist ersichtlich, dass auch eine Kombination aus mehreren Sensoren, nämlich beispielsweise ein kapazitiver Sensor 9, ein induktiver Sensor 11 und ein resistiver Sensor 13, innerhalb einer Schicht gedruckt werden kann.

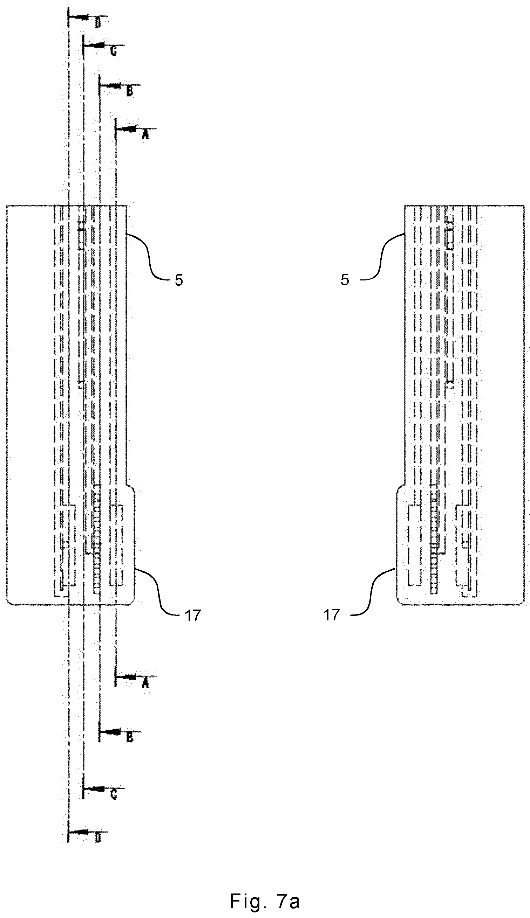

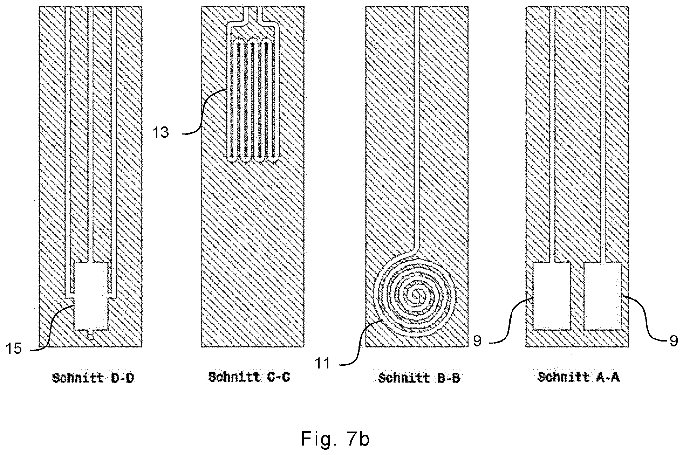

Statt mehrere gleichartige oder auch unterschiedliche Sensoren 9, 11, 13, 15 sowie beliebige Kombinationen daraus innerhalb einer Schicht zu drucken, ist es, wie in dem aus Fig. 7a und Fig. 7b ersichtlichen sechsten Ausführungsbeispiel dargestellt, auch möglich, Sensoren 9, 11, 13, 15 über unterschiedliche Schichten zu verteilen. Statt wie in Fig. 7b, rechte Abbildung (Schnitt A-A) dargestellt, können die beiden in der Zeichenebene horizontal nebeneinander liegenden Platten eines kapazitiven Sensors 9 selbstverständlich auch senkrecht übereinander oder versetzt zueinander angeordnet sein. Die in Schnitt B-B dargestellte kreisförmige, schematische Ausbildung der Spule 11 steht nur beispielhaft für eine beliebige spiralförmige, insbesondere eckförmige Ausbildung.

Durch den schichtweisen Aufbau mittels Multimaterialdruck (3D) ist es zudem möglich, verschiedene Trägermaterialien zu kombinieren. Beispielsweise können härtere Trägermaterialen in von Greifflächen (senkrecht zur Fingerlängsachse L) entfernten Schichten verwendet werden, um die Stabilität eines Greifelements, insbesondere Fingers 5 zu erhöhen. Dagegen können im Bereich von Greifflächen 17, insbesondere in den Endbereichen (Fingerspitzen), für den Druck einer äußeren Schicht (und evtl. zusätzlich der darunter liegenden Schichten) weiche, elastische Materialen, wie beispielsweise Gummi, verwendet werden, um die Reibung zwischen Finger 5 und Objekt zu erhöhen und Beschädigungen an einem zu greifenden Objekt zu vermindern.

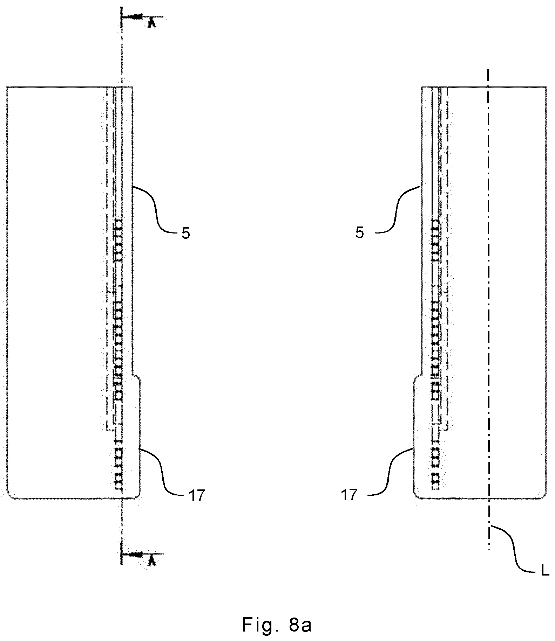

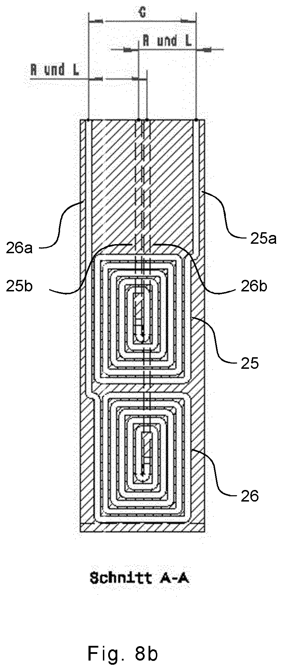

Das in Fig. 8a und Fig. 8b dargestellte sechste Ausführungsbeispiel zeigt einen Multisensor, insbesondere 4-in-1-Sensor, mit zwei in der Zeichenebene untereinander angeordneten rechteckförmig ausgebildeten Spiralen 25 (erstes Multisensorelement) und 26 (zweites Multisensorelement). Je nachdem, wie die Spiralen 25 und 26 über ihre Anschlüsse 25a, b und 26a, b angeschlossen bzw. verschaltet werden, lässt sich dieser Multisensor als induktiver, kapazitiver oder resistiver Sensor (DMS) betreiben.

Werden die beiden Endabgriffe 25a und 26a (oder 25b und 26b) verwendet, so wirkt der Multisensor als kapazitiver Sensor, wobei die beiden Sensorelemente 25 und 26 als Kondensatorplatten dienen.

Werden nur die äußeren und inneren Anschlüsse bzw. Abgriffe 25a und 25b bzw. 26a und 26b verwendet, wirkt jedes Sensorelement 25 und 26 als resistiver oder induktiver Sensor. Statt einer doppelten Ausbildung dieser Sensoren ist es selbstverständlich auch denkbar, die Sensorelemente in Reihe zu schalten, so dass die Sensorelemente als ein vergrößerter induktiver oder resistiver Sensor wirken.

Wirken die Sensorelemente 25 und 26 als zwei Sensoren, können die beiden erhaltenen Messwerte zur Gegenprüfung (einfache Redundanz) oder zum Eliminieren von Störfaktoren (beispielsweise Eliminierung des Einflusses von Erwärmung in ein Messergebnis) verwendet werden.

### Bezugszeichenliste:

- 1: Greifsystem
- 3: Steuerungseinheit
- 5: Finger
- 7: Sensor
- 7a: Zuleitung des Sensors 7
- 9: Kondensatorplatten bzw. kapazitiver Sensor
- 9a: Zuleitung des Sensors 9
- 11: Spulen bzw. induktiver Sensor
- 11a: Zuleitung des Sensors 11 äußerer Anschluss (Endabgriff)
- 11b: Zuleitung des Sensors 11 innerer Anschluss (Mittelabgriff)
- 13: Dehnungsmessstreifen bzw. resistiver Sensor
- 13a: Sensorzuleitung
- 15: Hall-Sensoren
- 15a: Sensorzuleitung
- 17: Greiffläche
- 19: Kamera
- 21: Objektiv
- 23: Faserverstärkung
- 25: erstes Multisensorelement
- 25a: Zuleitung äußerer Anschluss (Endabgriff) des Sensors 25
- 25b: Zuleitung innerer Anschluss (innerer Abgriff) des Sensors 25
- 26: zweites Multisensorelement
- 26a: Zuleitung äußerer Anschluss (Endabgriff) des Sensors 26
- 26b: Zuleitung innerer Anschluss (innerer Abgriff) des Sensors 26
- M: Mittelachse des Greifsystems 1
- L: Längsachse des Fingers 5
- Z: Detail

## Patentansprüche

1. Verfahren zum 3D-Druck eines Roboterelements, nämlich eines Greifbackens, insbesondere eines Fingers (5), zum Einsatz in der Robotik,
bei dem mittels Multimaterialdruck wenigstens ein Sensor (7) während des Drucks des Greifbackens mitgedruckt wird.

2. Verfahren nach Anspruch 1, **dadurch gekennzeichnet, dass** eine Kombination von mehreren Sensoren (9, 11, 13, 15) während des Drucks des Greifbackens mitgedruckt wird.

3. Verfahren nach Anspruch 1 oder 2, **dadurch gekennzeichnet, dass** Anschlüsse, Leitungen (7a, 9a, 11a, b, 13a, 15a, 25a, b, 26a, b), Stecker und/oder Verbindungselemente mitgedruckt werden.

4. Verfahren nach einem der vorhergehenden Ansprüche, **dadurch gekennzeichnet, dass** Verbundmaterial, insbesondere Faserverstärkung, mitgedruckt oder eingebettet wird.

5. Verfahren nach einem der vorhergehenden Ansprüche, **dadurch gekennzeichnet, dass** Sensoren (9, 11, 13, 15) und Faserverstärkung während des Drucks auf unterschiedliche Ebenen verteilt werden.

6. Verfahren nach einem der vorhergehenden Ansprüche, **dadurch gekennzeichnet, dass** in dem Greifbacken wenigstens eine Kamera (19) durch Einbetten während des Drucks integriert wird.

7. Verfahren nach einem der vorhergehenden Ansprüche, **dadurch gekennzeichnet, dass** ein gesamter Finger (5) in einem Stück gedruckt wird.

8. Greifbacken, insbesondere Finger (5) für einen Roboter, hergestellt durch ein Verfahren nach einem der vorhergehenden Ansprüche.

## Claims

1. Method for 3D printing a robot element, namely a gripping jaw, in particular a finger (5), for use in robotics,
wherein, by means of multi-material printing, at least one sensor (7) is co-printed during the printing of the gripping jaw.

2. Method according to claim 1, **characterized in that** a combination of a plurality of sensors (9, 11, 13, 15) is co-printed during the printing of the gripping jaw.

3. Method according to claim 1 or claim 2, **characterized in that** connections, lines (7a, 9a, 11a, b, 13a, 15a, 25a, b, 26a, b), plugs and/or connecting elements are co-printed.

4. Method according to any of the preceding claims, **characterized in that** composite material, in particular fiber reinforcement, is co-printed or embedded.

5. Method according to any of the preceding claims, **characterized in that** the sensors (9, 11, 13, 15) and the fiber reinforcement are distributed across different planes during printing.

6. Method according to any of the preceding claims, **characterized in that** at least one camera (19) is integrated in the gripping jaw by means of embedding during printing.

7. Method according to any of the preceding claims, **characterized in that** an entire finger (5) is printed in a single piece.

8. Gripping jaw, in particular a finger (5), for a robot, which gripping jaw is manufactured by means of a method according to any of the preceding claims.

## Revendications

1. Procédé pour l'impression 3D d'un élément pour robot, à savoir une mâchoire de préhension, en particulier un doigt (5), destiné à être utilisé en robotique,
dans lequel, au moyen d'une impression multimatériaux, au moins un capteur (7) est imprimé pendant l'impression de la mâchoire de préhension.

2. Procédé selon la revendication 1, **caractérisé en ce qu'**une combinaison de plusieurs capteurs (9, 11, 13, 15) est imprimée pendant l'impression de la mâchoire de préhension.

3. Procédé selon la revendication 1 ou 2, **caractérisé en ce que** des raccords, des lignes (7a, 9a, 11a, b, 13a, 15a, 25a, b, 26a, b), des fiches et/ou des éléments de liaison sont également imprimés.

4. Procédé selon l'une des revendications précédentes,
**caractérisé en ce qu'**un matériau composite, en particulier un renforcement de fibres, est imprimé ou incorporé.

5. Procédé selon l'une des revendications précédentes,
**caractérisé en ce que** les capteurs (9, 11, 13, 15) et le renforcement de fibres sont répartis sur différents plans pendant l'impression.

6. Procédé selon l'une des revendications précédentes,
**caractérisé en ce qu'**au moins une caméra (19) est intégrée dans la mâchoire de préhension par incorporation lors de l'impression.

7. Procédé selon l'une des revendications précédentes,
**caractérisé en ce qu'**un doigt (5) entier est imprimé en une seule pièce.

8. Mâchoire de préhension, en particulier doigt (5) pour un robot, fabriquée par un procédé selon l'une des revendications précédentes.
